# EUROPEAN PATENT SPECIFICATION

(11) **EP 2 012 698 B1**
(45) Date of publication and mention of the grant of the patent: **02.03.2011**
(21) Application number: 07724146.1
(22) Date of filing: 11.04.2007
(51) Int. Cl.: A61B 19/00

(54) **Interactive virtual mirror device for visualizing virtual objects in endoscopic applications**
Interaktive virtuelle Spiegelvorrichtung zur Visualisierung von virtuellen Objekten in endoskopischen Anwendungen
Dispositif miroir virtuel interactif destiné à la visualisation d'objets virtuels dans des applications endoscopiques

(30) Priority: 04.05.2006 EP 06009221
(43) Date of publication of application: 14.01.2009
(73) Proprietor: NAVAB, Nassir, 81247 München (DE)
(72) Inventor: BICHLMEIER, Christoph, 80469 München (DE); FEUERSTEIN, Marco, 80333 München (DE)
(86) International application number: PCT/EP2007/003205
(87) International publication number: WO 2007/128377

(56) References cited:
- WO-A-02/100284
- WO-A1-00/56215
- US-A1- 2001 007 919
- US-B1- 6 711 433

## Description

The present invention relates to an augmented reality device as defined in claim 1.

A major roadblock for using augmented reality in many medical and industrial applications is the fact that the user cannot take full advantage of the 3D virtual data. This usually requires the user to move the virtual object, which disturbs the real/virtual alignment, or to move his head around the real objects, which is not always possible and/or practical. This problem becomes more dramatic when a single camera is used for monitor based augmentation, such as in augmented endoscopic surgery.

When augmenting a monoscopic endoscope, a 3D volume is projected onto the endoscope's image plane, so one dimension is totally lost, leading to even more limited perception of 3D shape and depth during superimposition. However, particularly for interventions targeting the inside of organs 3D shape information is crucial, for instance when identifying vessel bifurcations, which can be ambiguous due to overlaps and occlusions in 2D.

Document US 2001/0 007 919 A1 discloses an augmented reality device according to the preamble of claim 1.

EP06007724.5 relates to a method and to a virtual penetrating mirror device for visualizing virtual objects from arbitrary view points within an augmented reality environment, with substantial usefulness in medical and industrial applications.

EP06009222.8 relates to a method and to a device, which is a registration-free augmentation device, for collocating the view of a tracked endocope with an intraoperative reconstruction of anatomy for image guided surgery using an endoscope and a C-arm or another imaging device capable of intraoperative 3D or 4D reconstructions of anatomy with one or more co-registered tracking systems to localize the endoscope, the imaging device and its reconstructions, as well as additional surgical instruments in a common coordinate system.

However neither EP06007724.5 nor EP06009222.8 discloses any practical use of the mentioned devices and methods for the medical applications. In addition, they do not perceive possible extensions of the two inventions in order to provide a new solution for intraoperative reconstruction and 3D visualization of the said reconstruction using an interactive penetrating virtual mirror, reflecting the reconstructed shape as well as other endoscopic instruments.

The objective of the present invention is to remove the lack of ability of taking full advantage of the 3D virtual data for using augmented reality in endoscopic applications and the lack of shape information for endoscopic interventions targeting the inside of organs.

The invention achieves this objective using a virtual penetrating mirror device for visualizing virtual objects for endoscopy by proper application methods for interactive visualization of the augmented 3D data on a traditional endoscopic display or on the display of a monocular optical or video see-through augmented reality system. In particular, this invention focuses on providing a 3D interactive visualization method for a monitor based augmented reality system. In such systems, at each moment the user observes the real world from one viewpoint. When the real scene is augmented with aligned virtual data, the user's view is still two dimensional. In order to leverage the 3D nature of the augmented data, the existing interaction paradigms are not satisfactory. They usually require the user to displace or rotate the 3D object out of the alignment or to move around the real object, which is often not possible or practical.

This is the case in many computer aided surgery applications, where the surgeon cannot move freely to observe the virtual data from arbitrary viewpoints.

In a preferred embodiment of this invention, an optically tracked cone-beam CT capable mobile C-ann is used during the intervention in a medical application to reconstruct a high-resolution 3D volume of the target region. This volume is directly augmented on the live video of a tracked endoscope.

This method can be exemplarily applied, where it is essential to the surgeon to identify the correct vessels to be clipped in order to avoid bleeding, when for instance performing a liver resection.

After patient positioning, placement of the trocars, and insufflation of carbon dioxide, a iodinated nonionic contrast agent is administered to the patient. A C-arm acquisition is started immediately and synchronized with the contrast agent's penetration into the liver vessels. The contrasted liver vessels are clearly visible in a reconstructed volume. Having C-arm and endoscope calibrated as well as registered in a common coordinate frame, the contrasted vessels can be directly augmented on the endoscopic video.

For interventions targeting the inside of organs 3D shape information is crucial, for instance when identifying vessel bifurcations, which can be ambiguous due to overlaps and occlusions in 2D.

To recover this lost shape information a virtual mirror is used, which is placed inside the augmented view. The endoscopic Virtual Mirror is able to virtually reflect the 3D volume as well as the endoscope or any other involved instruments, which are modelled and tracked. When dexterously placing the Endoscopic Virtual Mirror on the side of the target region, missing shape information can be revealed through the perceptually familiar concept of a reflection onto the mirror. As the Endoscopic Virtual Mirror can also be manipulated by a tracked interaction device, the user could move it within the personal space. The visual feedback provided by the reflection depends on exact position and motion of the endoscope and the mirror. Therefore, the observer senses spatial information of the objects through proprioception. This information is gathered from stimuli of sensory receptors, the so called proprioceptors, found in muscles, tendons and joint capsules and generates sensation about the observer's position in relation to his/her spatial environment.

This additional sensory information allows the user to better perceive the augmented 3D volume, its depth, exact dimensions, and complex structures. This new concept presents itself as an attractive alternative to more complex solutions based on stereo view and 3D displays.

To precisely guide the surgical staff to regions inside a specific organ, e.g. blood vessels to be clipped for tumour resection, a high-resolution intra-operative imaging data generated by a mobile C-arm with cone-beam CT imaging capability could be used. Both the C-arm and the endoscope are tracked and registered in a common world coordinate frame. After patient positioning, port placement, and carbon dioxide insufflation, a C-arm volume is reconstructed during patient exhalation and superimposed in real time on the endoscopic live video without any need of time-consuming or imprecise patient registration steps.

To overcome the missing perception of 3D depth and shape when rendering virtual volume data directly on top of the organ's surface view, the method of a endoscopic virtual mirror is applied, which uses a virtual reflection plane augmented on the endoscopic live video that is able to visualize a reflected side view of the organ and its interior. This enables the surgical staff to observe the 3D structure of for example blood vessels by moving the virtual mirror within the augmented monocular view of the endoscope.

The invention will now be elucidated by reference to the embodiment partially illustrated schematically in the drawings regarding an exemplary augmented reality scenario for endoscopic surgery using a favourable hardware set-up:
Fig. 1: a schematic view of an exemplary augmented reality scene with required hardware set up
Fig. 2: a schematic view of an exemplary tracked endoscopic camera 2
Fig. 3: a schematic view of an exemplary tracked endoscopic instrument 3
Fig. 4: a schematic overview to get a view onto a display for image of endoscope 24

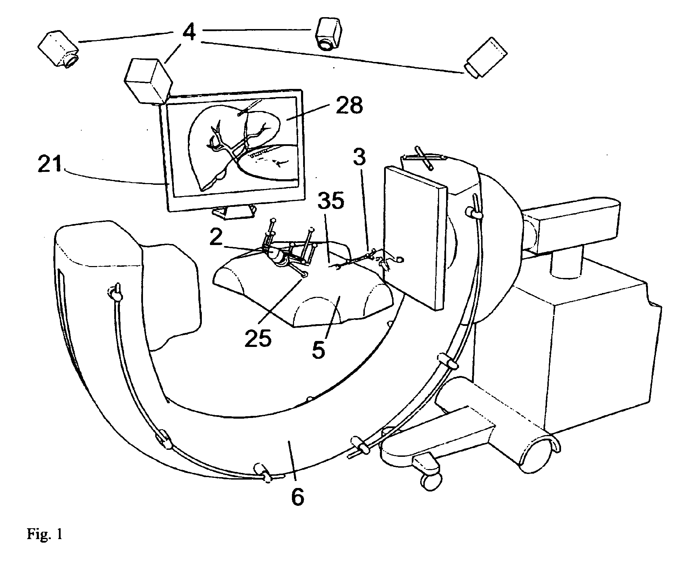

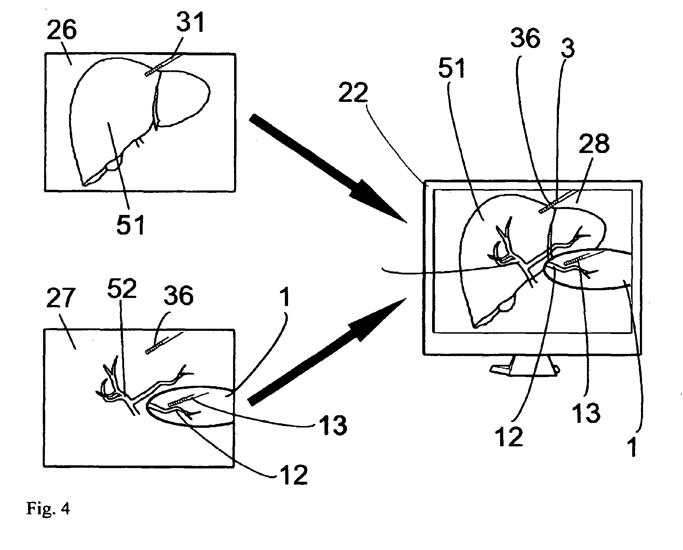

Fig. 1 shows an exemplary augmented reality scenario including a required hardware set up having an interactive virtual mirror 1, a 3D or 4D medical image of patient's anatomy 52 (shown in Fig. 4), a displaying device 21 and a display of displaying device 22, tracking system 4 and a tracked endoscopic camera 2, providing a endoscopic, augmented reality view on display of display device 22. A device for medical imaging data acquisition 6 provides medical imaging data of a body 5 to visualize 3D virtual image of anatomy 52. The tracked endoscopic camera 2 tracked by the tracking system 4 is inserted into the body 5 through the trocar for tracked endoscope 25 to capture video images of patient anatomy 51. Endoscopic, real view on display 26 is combined with endoscopic virtual view on display 27 to generate the endoscopic augmented reality view on display 28.

At least one additional tracked endoscopic instrument 3 localized by the tracking system 4 is inserted through a trocar for tracked endoscopic instrument 35.

The interactive virtual mirror 1 is presented in a common coordinate system with the 3D or 4D medical image of patient's anatomy 52 and provides full integration and combined visualization of the reconstruction of the 3D or 4D medical image of patient's anatomy 52, the endoscopic image 26 and the virtual mirror 1 on the display of displaying device 22 and hence providing additional perspectives on the reconstructed 3D or 4D medical image of patient's anatomy 52 within the view of endoscopic image 26.

The 3D virtual image of anatomy 52 (shown in Fig. 4) is reconstructed intraoperatively in the same coordinate system as the tracking system 4 by a tracked device for medical imaging data acquisition 6.

The 3D virtual image of anatomy 52 can be reconstructed after a contrast injection.

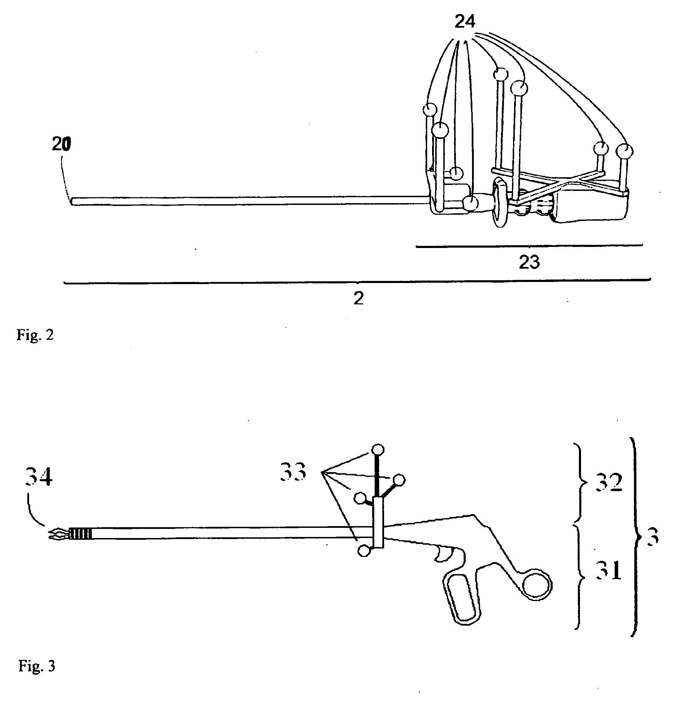

Fig. 2 shows a tracked endoscopic camera 2 consisting of a camera 20 a tracking target of endoscope 23. The tracking target of endoscope 23 consists of a set of markers of endoscope 24 to be tracked by the tracking system 4.

The tracked virtual mirror 1 can virtually be attached to the tracked endoscopic camera 2 and be interacted by the tracked endoscopic camera 2.

Fig. 3 shows an exemplary tracked endoscopic instrument 3 consisting of the endoscopic instrument 31 and a tracking target of endoscope instrument 32. The tracking target of endoscopic instrument 32 consists of a set of markers of endoscopic instrument 33 to be tracked by the tracking system 4. When the tracked endoscopic instrument 3 is inserted into the body 5, in some cases the tip of the endoscopic instrument 34 can be also be seen in the endoscopic, augmented reality view on display 28.

The tracked virtual mirror 1 can virtually be attached to the tracked endoscopic instrument 3 and be interacted by the tracked endoscopic instrument 3.

Fig. 4 shows the endoscopic, real view on display 26, the endoscopic, virtual view on display 27 and the combination of both, the endoscopic, augmented reality view on display 28 including the virtual mirror 1 on a display of displaying device 22. The endoscopic, real view on display 26 shows the patient anatomy 51 inside the body 5 and the endoscopic instrument 31. The endoscopic, virtual view on display 27 shows the 3D virtual image of anatomy 52 inside the body 5, the virtual, tracked endoscopic instrument 36 and the virtual mirror 1 with the image of the reflected virtual object by virtual mirror on display 12 and the image of the reflected virtual, tracked endoscopic instrument by virtual mirror on display 13.

The tracked virtual mirror 1 can virtually be guided by any interaction device like a computer mouse, keyboard and steering wheels.

### Reference list of drawings

- 1: tracked virtual mirror
- 12: image of the reflected virtual object by virtual mirror on display
- 13: image of the reflected virtual, tracked endoscopic instrument by virtual mirror on display
- 2: tracked endoscopic camera
- 21: display device
- 22: display of displaying device
- 23: tracking target of endoscope
- 24: markers of endoscope
- 25: trocar for tracked endoscope
- 26: endoscopic, real view on display
- 27: endoscopic, virtual view on display
- 28: endoscopic, augmented reality view on display
- 3: tracked endoscopic instrument
- 31: endoscopic instrument
- 32: tracking target of endoscopic instrument
- 33: markers of endoscopic instrument
- 34: tip of endoscopic instrument
- 35: trocar for tracked endoscopic instrument
- 36: virtual, tracked endoscopic instrument
- 4: tracking system
- 5: body
- 51: patient anatomy
- 52 3D or 4D: medical image of patient's anatomy
- 6: device for medical imaging data acquisition

## Claims

1. An augmented reality device for visualizing virtual objects in endoscopic applications, which comprises
a displaying device (21),
a display of displaying device (22),
a tracking system (4), and
a tracked endoscopic camera (2),
wherein the display of displaying device (22) is adapted to visualize a reconstruction of a 3D or 4D medical image of a patient's anatomy (52)
**characterized in that**
the display of displaying device (22) is further adapted to visualize an interactive virtual mirror (1), which is presented in a common coordinate system with the reconstruction of the 3D or 4D medical image of the patient's anatomy (52), and
the display of displaying device (22) provides full integration and combined visualization of the reconstruction of the 3D or 4D medical image of the patient's anatomy (52), the endoscopic image (26) of said tracked endoscopic camera and the virtual mirror (1).

2. The augmented reality device according to claim 1,
**characterized in that**
a tracked endoscopic instrument (3) is also reflected by the virtual mirror (1).

3. The augmented reality device according to any one of the preceding claims,
**characterized in that**
the 3D or 4D medical image of the patient's anatomy (52) is reconstructed intraoperatively in the same coordinate system as the tracking system (4) by a tracked device for acquisition of medical imaging data (6).

4. The augmented reality device according to any one of the preceding claims,
**characterized in that**
the device is adapted to reconstruct the 3D or 4D medical image of the patient's anatomy (52) after contrast injection.

5. The augmented reality device according to any one of the preceding claims,
**characterized in that**
the display is adapted to visualize the virtual mirror (1) being virtually attached to the tracked endoscopic camera (2).

6. The augmented reality device according to any one of the preceding claims,
**characterized in that**
the position, orientation, size, scaling and color of the virtual mirror (1) is controllable -interactively.

## Patentansprüche

1. Vorrichtung für erweiterte Realität (Augmented Reality) zur Visualisierung von virtuellen Objekten in endoskopischen Anwendungen, die aufweist eine Anzeigevorrichtung (21),
eine Anzeige der Anzeigevorrichtung (22),
ein Trackingsystem (4), und
eine getrackte endoskopische Kamera (2),
wobei die Anzeige der Anzeigevorrichtung (22) eingerichtet ist, eine 3D- oder 4D-Rekonstruktion eines medizinischen Bildes einer Patientenanatomie (52) zu visualisieren, **dadurch gekennzeichnet, dass**
die Vorrichtung einer Anzeigevorrichtung (22) ferner eingerichtet ist, einen interaktiven virtuellen Spiegel (1) zu visualisieren, der in einem gemeinsamen Koordinatensystem mit der 3D- oder 4D-Rekonstruktion des medizinischen Bildes der Patientenanatomie (52) dargestellt ist, und
die Anzeige der Anzeigevorrichtung (22) vollständige Integration und kombinierte Visualisierung der 3D- oder 4D-Rekonstruktion des medizinischen Bildes der Patientenanatomie (52), des endoskopischen Bildes (26) der getrackten endoskopischen Kamera und des virtuellen Spiegels (1) liefert.

2. Vorrichtung für erweiterte Realität nach Anspruch 1,
**dadurch gekennzeichnet, dass**
ein getracktes endoskopisches Instrument (3) auch durch den virtuellen Spiegel (1) reflektiert wird.

3. Vorrichtung für erweiterte Realität nach einem der vorhergehenden Ansprüche,
**dadurch gekennzeichnet, dass**
das medizinische 3D-oder 4D-Bild der Patientenanatomie (52) in dem gleichen Koordinatensystem intraoperativ durch eine getrackte Vorrichtung zum Erwerb medizinischer Bilddaten (6) rekonstruiert wird wie das Trackingsystem (4).

4. Vorrichtung für erweiterte Realität nach einem der vorhergehenden Ansprüche,
**dadurch gekennzeichnet, dass**
die Vorrichtung eingerichtet ist, das medizinische 3D- oder 4D-Bild der Patientenanatomie (52) nach einer Kontrastmittelinjektion zu rekonstruieren.

5. Vorrichtung für erweiterte Realität nach einem der vorhergehenden Ansprüche,
**dadurch gekennzeichnet, dass**
die Anzeige eingerichtet ist, den virtuellen Spiegel (1), der virtuell an der getrackten endoskopischen Kamera (2) angebracht ist, zu visualisieren.

6. Vorrichtung für erweiterte Realität nach einem der vorhergehenden Ansprüche,
**dadurch gekennzeichnet, dass**
die Position, Orientierung, Größe, Skalierung und Farbe des virtuellen Spiegels (1) interaktiv gesteuert werden kann.

## Revendications

1. Un dispositif de réalité augmentée (Augmented Reality) pour visualiser des objets virtuels dans des applications endoscopiques, comprenant
un dispositif d'affichage (21),
un affichage d'un dispositif d'affichage (22),
un système de traçage (4) et
une caméra endoscopique tracée (2),
dans lequel l'affichage du dispositif d'affichage (22) est adapté afin de visualiser une reconstruction d'une image médicale 3D ou 4D de l'anatomie d'un patient (52) ;
**caractérisé en ce que**
l'affichage du dispositif d'affichage (22) est en outre adapté afin de visualiser un miroir virtuel interactif (1), qui est présenté dans un système commun de coordonnées avec la reconstruction de l'image médicale 3D ou 4D de l'anatomie du patient (52), et
l'affichage du dispositif d'affichage (22) fournit intégration complète et visualisation combinée de la reconstruction de l'image médicale 3D ou 4D de l'anatomie du patient (52), l'image endoscophique (26) de ladite caméra endoscopique tracée et le miroir virtuel (1).

2. Le dispositif de réalité augmentée selon la revendication 1,
**caractérisé en ce que**
l'instrument endoscopique tracé (3) est aussi reflété par le miroir virtuel (1).

3. Le dispositif de réalité augmentée selon l'une quelconque des revendications précédentes,
**caractérisé en ce que**
l'image médicale 3D ou 4D de l'anatomie du patient (52) est reconstruite intraopérativement dans le même système de coordonnées que le système de traçage (4) par un dispositif tracé pour acquisition de données d'imagination médicales (6).

4. Le dispositif de réalité augmentée selon l'une quelconque des revendications précédentes,
**caractérisé en ce que**
le dispositif est adapté afin de reconstruire l'image médicale 3D ou 4D de l'anatomie du patient (52) après injection d'une substance de contraste.

5. Le dispositif de réalité augmentée selon l'une quelconque des revendications précédentes,
**caractérisé en ce que**
l'affichage est adapté afin de visualiser le miroir virtuel (1) étant rattaché virtuellement à la caméra endoscopique tracée (2).

6. Le dispositif de réalité augmentée selon l'une quelconque des revendications précédentes,
**caractérisé en ce que**
la position, l'orientation, la taille, le redimensionnement et la couleur du miroir virtuel (1) peuvent être contrôlés interactivement.
